# EUROPEAN PATENT SPECIFICATION

(11) **EP 1 661 520 B1**
(45) Date of publication and mention of the grant of the patent: **15.07.2009**
(21) Application number: 05026140.3
(22) Date of filing: 30.11.2005
(51) Int. Cl.: A61B 10/00

(54) **Skin punch instrument**
Hautstanzgerät
Instrument pour la perforation de la peau

(30) Priority: 30.11.2004 JP 2004347474
(43) Date of publication of application: 31.05.2006
(73) Proprietor: KAI R&D CENTER CO., LTD., Seki-shi Gifu-ken 501-3992 (JP)
(72) Inventor: Endo, Masahiro, Seki-shi Gifu-ken 501-3992 (JP)
(74) Representative: Hinkelmann, Klaus

(56) References cited:
- EP-A- 0 536 888
- US-A- 3 522 809
- US-A- 3 692 020
- US-A1- 2004 193 203

## Description

The present invention relates to skin punch instruments used for removing a portion of the skin for purposes like medical examination.

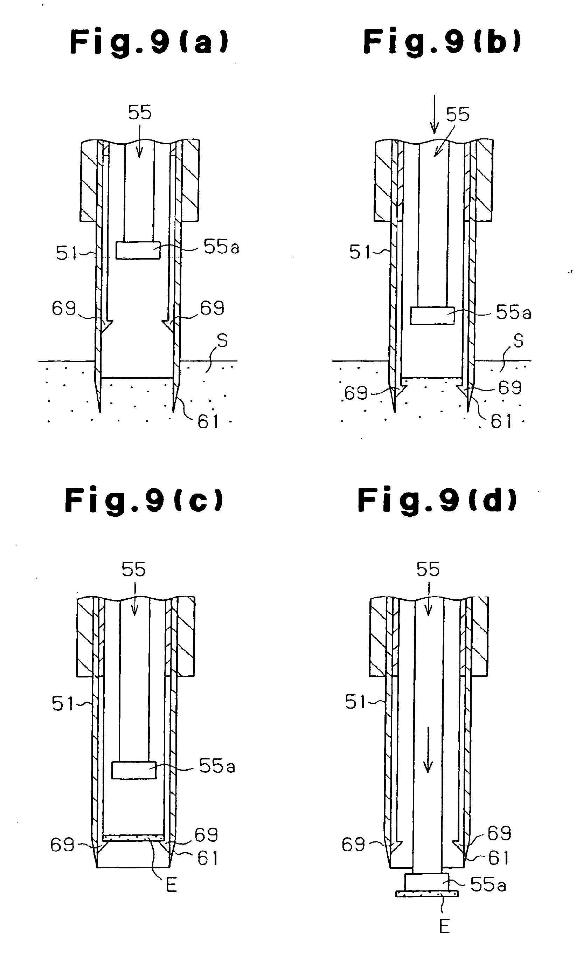

Using a skin punch instrument described in Japanese Laid-Open Patent Publication No. 2000-126196, a portion of the skin S is removed in accordance with the steps shown in Figs. 9(a), 9(b), and 9(c). More specifically, after holding a sample E by opposed cutting ends 69 of a removal blade 53 in the interior of a blade sleeve 51, a manipulation body (not shown) is depressed together with a pressing body 55 against elastic force of a compression coil spring (not shown), as illustrated in Fig. 9(d). A contact portion 55a of the pressing body 55 is thus projected from a sample outlet 61 of the blade sleeve 51. In this manner, the sample E is separated from the cutting ends 69 and pressed out of the sample outlet 61.

However, if the manipulation body is released prematurely, the elastic force of the compression coil spring acts to return the pressing body 55 from the sample outlet 61 to the interior of the blade sleeve 51, thus restoring the state of Fig. 9(c). At this stage, if the sample E is still held by the contact portion 55a, the sample E is also returned into the interior of the blade sleeve 51. If this is the case, the manipulation body and the pressing body 55 must be forcibly re-depressed against the force of the coil spring. Then, with the manipulation body held in the forcibly depressed state, the sample E must be removed from the contact portion 55a of the pressing body 55, which complicates the sample taking operation.

Accordingly, it is an objective of the present invention to facilitate skin removing operation using a skin punch instrument having a pressing body.

US-A-3 692 020 discloses a rotary punch for excising uniform biopsy specimens, comprising:
a. a housing;
b. a hollow cylindrical spindle longitudinally disposed within the housing;
c. a torque spring axially aligned over the external surface of the spindle;
d. a winding knob for storing torque in the spring, the knob being circumferentially secured to the spindle
e. a cutting die assembly comprising:
   1. a cutting die for excising a biopsy specimen threaded onto the forward end of the spindle;
   2. a die guard sleeve covering and coaxially aligned with the cutting die;
   3. a compression spring for holding the die guard sleeve in place; and
   4. a cutting die depth control collar threaded on the sleeve for regulating the penetrating depth of the cutting die;
f. an actuating mechanism; and
g. a specimen ejector mechanism for ejecting biopsy specimens from the cutting die.

To achieve the foregoing and other objectives and in accordance with the purpose of the present invention, the invention provides a skin punch instrument according to claim 1 having a blade sleeve including an annular cutting edge formed at a distal end of a handle. A pressing body is received in the blade sleeve in an axially movable manner. A contact portion is formed at a distal end of the pressing body. The contact portion may be located at a retracted position separated inwardly from the cutting edge of the blade sleeve or a projected position separated from the retracted position toward the cutting edge of the blade sleeve. The skin punch instrument includes a manipulation member for moving the pressing body between the retracted position and the projected position. The skin punch instrument also includes a projected position holding member for holding the pressing body at the projected position.

Other aspects and advantages of the invention will become apparent from the following description, taken in conjunction with the accompanying drawings, illustrating by way of example the principles of the invention.

The invention, together with objects and advantages thereof, may best be understood by reference to the following description of the presently preferred embodiments together with the accompanying drawings in which:
Fig. 1(a) is a front view showing a skin punch instrument according to a first embodiment of the present invention with a pressing body retracted;
Fig. 1(b) is a cross-sectional view taken along line 1b-1b of Fig. 1(a);
Fig. 2(a) is a front view showing the skin punch instrument with the pressing body projected;
Fig. 2(b) is a cross-sectional view taken along line 2b-2b of Fig. 2(a);
Figs. 3(a), 3(b), 3(c) are enlarged cross-sectional views each showing a portion of the skin punch instrument for explaining a skin removing procedure using the skin punch instrument;
Fig. 4(a) is a front view showing a skin punch instrument according to a second embodiment of the present invention with a pressing body retracted;
Fig. 4(b) is a front view showing the skin punch instrument of Fig. 4(a) with the pressing body held in a state intermediate between a retracted state and a projected state;
Fig. 4(c) is a front view showing the skin punch instrument of Fig. 4(a) with the pressing body projected;
Fig. 5(a) is a cross-sectional view showing the pressing body in the retracted state of Fig. 4(a) as viewed from the front side;
Fig. 5(b) is a cross-sectional view showing the pressing body in the projected state of Fig. 4(c) as viewed from the front side;
Fig. 6(a) is a front view showing a skin punch instrument according to a modification of the second embodiment of the present invention with a pressing body retracted;
Fig. 6(b) is a front view showing the skin punch instrument of Fig. 6(a) with the pressing body held in a state intermediate between a retracted state and a projected state;
Fig. 6(c) is a front view showing the skin punch instrument of Fig. 6(a) with the pressing body projected;
Fig. 7(a) is an enlarged cross-sectional view showing a portion of a cutting edge of a blade sleeve of the skin punch instrument according to the first or second embodiment or the modification of the second embodiment;
Figs. 7(b) and 7(c) is an enlarged cross-sectional view showing a portion of a modified cutting edge of the blade sleeve;
Fig. 8 is a view showing a modification of a spring; and
Figs. 9(a), 9(b), 9(c), and 9(d) are views showing the operation steps of a prior-art skin punch instrument in order.

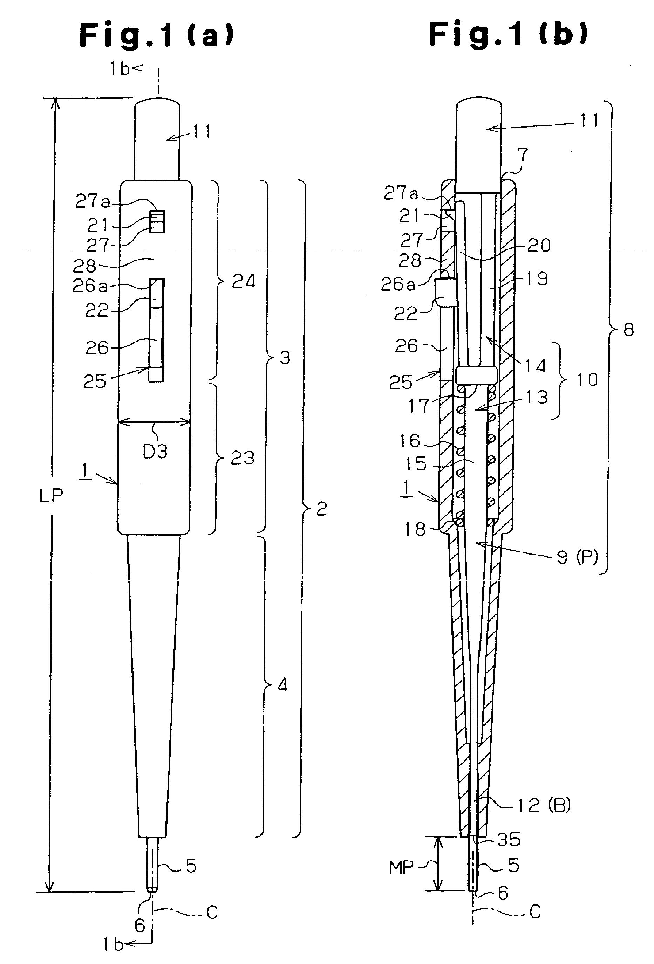

A skin punch instrument according to a first embodiment of the present invention will now be described with reference to Figs. 1(a) to 3(c).

A handle 1 includes a plastic handle body 2 having an upper sleeve section 3 and a lower sleeve section 4. A metal blade sleeve 5 is secured to the distal end of the lower sleeve section 4 and projects from the distal end of the lower sleeve section 4. An annular cutting edge 6 is formed at the distal end of the blade sleeve 5. The cutting edge 6 does not necessarily have to have a continuous annular shape extending along the entire circumference of the blade sleeve 5. That is, the cutting edge 6 may be formed in a noncontinuous shape including a slit extending along the axial direction C of the blade sleeve 5. An opening 7 is defined at the proximal end of the upper sleeve section 3 of the handle body 2. A pressing member 8 is inserted into the interior of the handle body 2 from the opening 7. The pressing member 8 is formed from plastic as an integral body, including a pressing body 9, a movable body 10, and a manipulation body 11. The movable body 10 and the manipulation body 11 form a manipulation member.

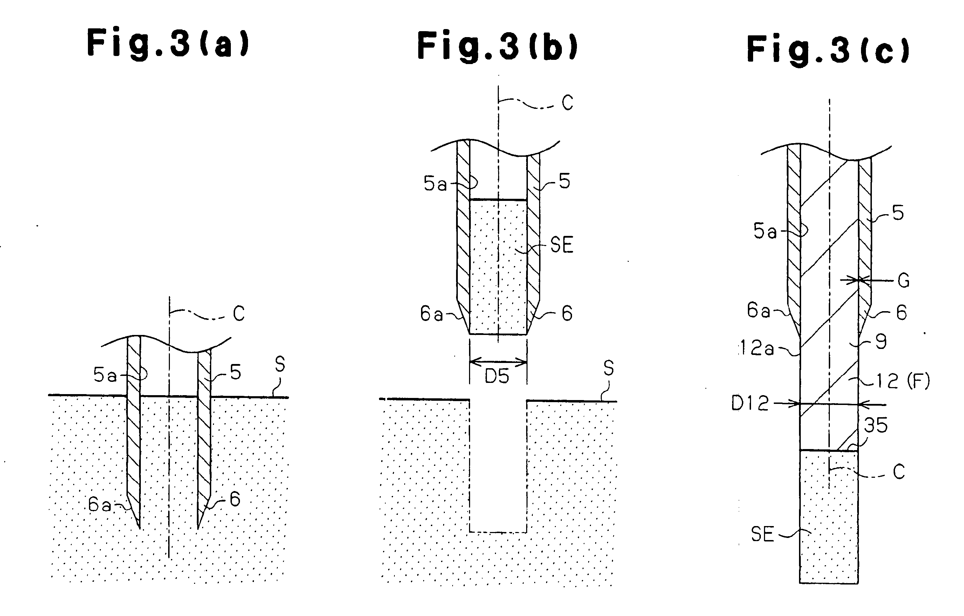

The pressing body 9 is formed as an elongated rod and has a columnar contact portion 12 formed at the distal end of the pressing body 9. The pressing body 9 is received in the lower sleeve section 4 of the handle body 2. The contact portion 12 of the pressing body 9 is received in the blade sleeve 5. As shown in Fig. 3(c), a gap G is defined between an inner circumferential portion 5a of the blade sleeve 5 including the cutting edge 6 and an outer circumferential portion 12a of the contact portion 12 of the pressing body 9. The movable body 10 is received in the upper sleeve section 3 of the handle body 2 and includes a spring support 13 and a connecting portion 14. The spring support 13 is connected with the pressing body 9. The connecting portion 14 connects the spring support 13 with the manipulation body 11. The manipulation body 11 is exposed from the opening 7 of the upper sleeve section 3 of the handle body 2 to the exterior. It is preferred that the gap G has a dimension of 0.01 mm to 5 mm, particularly 0.03 mm to 1 mm.

In correspondence with the spring support 13 of the movable body 10, a compression coil spring 16 (an elastic body) is wound around the outer circumference of a shaft portion 15 extending integrally from the proximal end of the pressing body 9. Referring to Fig. 1(b), an upper step 17 is formed at the boundary between the support 13 and the connecting portion 14 of the movable body 10. Similarly, a lower step 18 is formed at the boundary between the upper sleeve section 3 and the lower sleeve section 4 of the handle body 2. The upper step 17 supports the upper end of the compression coil spring 16 and the lower step 18 supports the lower end of the compression coil spring 16.

The connecting portion 14 of the movable body 10 includes a bridge 19 for connecting the shaft portion 15 of the spring support 13 integrally with the manipulation body 11. The connecting portion 14 also includes a leaf spring 20 (an elastic body) held in a cantilever manner adjacent to the bridge 19. The leaf spring 30 has a fixed end connected to the spring support 13 and a free end located in the vicinity of the manipulation body 11. An engagement projection 21 is formed at the free end of the leaf spring 20 and a manipulating projection 22 is formed at an intermediate portion between the free end and the fixed end of the leaf spring 20. The leaf spring 20 may be held at both ends by connecting the ends to the distal end and the proximal end of the bridge 19. Also, instead of the manipulating projection 22 of the movable body 10, a manipulating projection (not shown) for pressing the leaf spring 20 may be provided in the upper sleeve section 3 of the handle body 2.

The upper sleeve section 3 of the handle body 2 includes a holding portion 23 encompassing the spring support 13 of the movable body 10 and a manipulating portion 24 encompassing the connecting portion 14 of the movable body 10. A guide bore 25 is defined in the manipulating portion 24 of the upper sleeve section 3 and faces the leaf spring 20 of the connecting portion 14. The guide bore 25 includes a separating portion 28 for dividing the guide bore 25 into a first elongated hole 26 and a second elongated hole 27. The engagement projection 21 of the leaf spring 20 is engaged with the first or second elongated hole 26, 27 and thus exposed from the manipulating portion 24 to the exterior. The manipulating projection 22 is engaged with the first elongated hole 26 and thus exposed from the manipulating portion 24 to the exterior.

Figs. 1(a) and 1(b) each show a retracted state P of the pressing body 9. When the pressing body 9 is held in the retracted state P, the pressing member 8 is urged as a whole by the elastic force of the compression coil spring 16 toward the proximal end of the handle body 2. The manipulating projection 22 of the leaf spring 20 is thus moved along the first elongated hole 26 and toward the proximal end of the handle body 2. Meanwhile, the engagement projection 21 of the leaf spring 20 is engaged with the second elongated hole 27 and brought into contact with the proximal end of an inner wall 27a of the second elongated hole 27, on the movement path of the movable body 10. Further, the manipulation body 11 is projected from the opening 7 of the upper sleeve section 3 of the handle body 2. The contact portion 12 of the pressing body 9 is moved in the axial direction C of the blade sleeve 5 and retracted inwardly from the cutting edge 6 of the blade sleeve 5. The contact portion 12 is thus held at a retracted position B spaced from the cutting edge 6.

In the present embodiment, the manipulating projection 22 of the leaf spring 20 forms a disengagement portion of a retracted position holding member. The engagement projection 21 of the leaf spring 20 forms an engagement portion of the retracted position holding member. The proximal end of the inner wall 27a of the second elongated hole 27 also forms an engagement portion of the retracted position holding member.

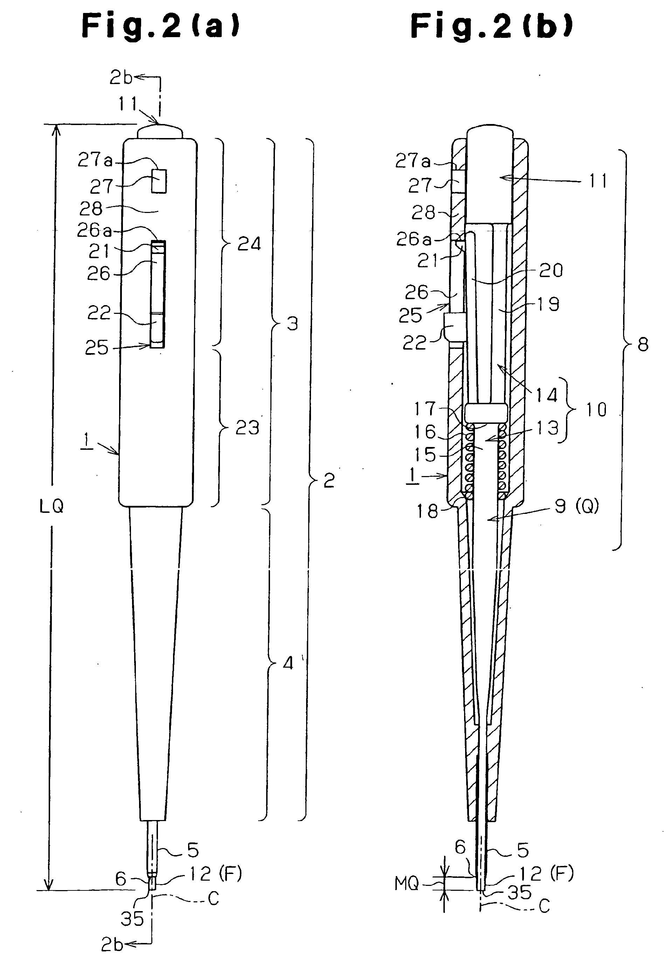

When the manipulation body 11 is depressed in the retracted state P, the pressing member 8 is moved as a whole toward the distal end of the handle body 2 against the elastic force of the compression coil spring 16 and switched to a projected state Q, as shown in Figs. 2(a) and 2(b). More specifically, the manipulating projection 22 of the leaf spring 20 is moved along the first elongated hole 26 and toward the distal end of the skin punch instrument. Meanwhile, the engagement projection 21 of the leaf spring 20 is urged by the elastic force of the leaf spring 20 to move away from the second elongated hole 27 and proceed along the separating portion 28, such that the engagement projection 21 is engaged with the first elongated hole 26. The engagement projection 21 is thus brought into contact with the proximal end of an inner wall 26a of the first elongated hole 26, on the movement path of the movable body 10. The contact portion 12 of the pressing body 9 is moved in the axial direction C of the blade sleeve 5 and held at a projected position F. At this position, the contact portion 12 is projected outwardly from the cutting edge 6 of the blade sleeve 5.

The manipulating projection 22 of the leaf spring 20 forms a disengagement portion of a projected position holding member. The engagement projection 21 of the leaf spring 20 forms an engagement portion of the projected position holding member. The proximal end of the inner wall 26a of the first elongated hole 26 also forms an engagement portion of the projected position holding member.

If the manipulating projection 22 of the leaf spring 20 is depressed in the projected state Q, the engagement projection 21 of the leaf spring 20 is separated from the proximal end of the inner wall 26a of the first elongated hole 26 and moved away from the first elongated hole 26. The engagement projection 21 thus proceeds along the separating portion 28 and is engaged with the second elongated hole 27. In this manner, the retracted state P is restored.

When the skin punch instrument is used, the cutting edge 6 of the blade sleeve 5 is first introduced into the skin S with the pressing body 9 maintained in the retracted state P, as illustrated in Fig. 3(a). The skin punch instrument is then rotated about the axis of the skin punch instrument and separated from the skin S, referring to Fig. 3(b). In this state, the sample SE may be caught in the blade sleeve 5. Thus, the pressing body 9 is switched to the projected state Q, as shown in Fig. 3(c), such that the sample SE is pressed out of the blade sleeve 5 by means of the contact portion 12 of the pressing body 9. The sample SE is then removed from the contact portion 12. The inner diameter D5 of the blade sleeve 5 may be set to 0.4 mm to 50 mm. If the inner diameter D5 of the blade sleeve 5 is as small as 5 mm or smaller, the sample SE is highly likely to be caught in the blade sleeve 5. In this case, the pressing body 9 is effectively used.

In the first embodiment, the inner diameter D5 of the blade sleeve 5 is set to 1.0 mm, the outer diameter D12 of the contact portion 12 is set to 0.9 mm, and the total length LP of the skin punch instrument held in the retracted state P is set to 107.3 mm, the total length LQ of the skin punch instrument held in the projected state Q is set to 99.8 mm, the projection amount MQ of the contact portion 12 from the cutting edge 6 is set to 1.7 mm, the retraction amount MP of the contact portion 12 from the cutting edge 6 is set to 7.5 mm, and the outer diameter D3 of the upper sleeve section 3 of the handle body 2 is set to 9.5 mm. It is preferred that the retraction amount MP is set to 1 mm to (D5 × 10) mm or 1 mm to 50 mm. It is also preferred that the projection amount MQ is set to 0.5 mm to (D5 × 2.5) mm or 0.5 mm to 20 mm. If the blade sleeve 5 defines an oval or rectangular cross-sectional shape, it is indicated that the inner diameter 5 corresponds to a maximum interval between opposing inner walls of the blade sleeve 5. The above-described numerals may be changed as needed.

Although the leaf spring 20 includes the engagement projection 21 and the manipulating projection 22, as has been described, the manipulating projection 22 may be omitted and the engagement projection 21 may function also as a manipulating projection. Further, the pressing member 8 includes the pressing body 9, the movable body 10, and the manipulation body 11 that are formed from plastic as an integral body. However, the pressing body 9 may be formed of metal separately from the movable body 10 and the manipulation body 11, which are formed of plastic as one body. In this case, the pressing body 9 is moved integrally with the movable body 10.

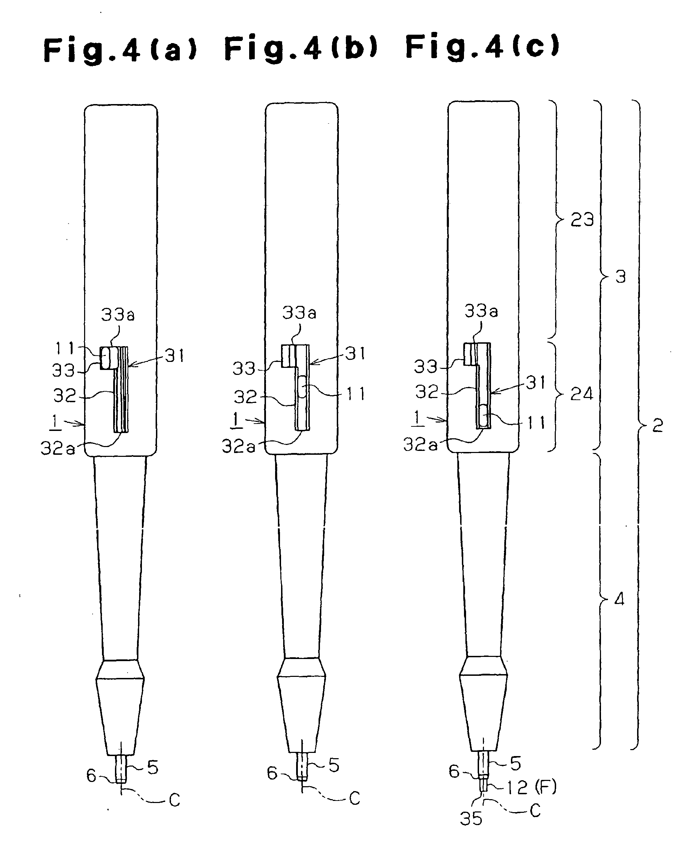

A skin punch instrument according to a second embodiment of the present invention will hereafter be described with reference to Figs. 4(a) to 5(b). The description focuses on the difference between the first and second embodiments. In the second embodiment, the movable body 10 and the manipulation body 11 of the first embodiment are modified as follows.

More specifically, a pair of leaf springs 30 (a pair of pressing contact portions each serving as an elastic body) are provided at opposing sides of the shaft portion 29 of the movable body 10, which extends from the proximal end of the pressing body 9. The leaf springs 30 are supported like cantilevers. A distal end 30a of each of the leaf spring 30 is pressed against and held in contact with an inner wall 3a of the upper sleeve section 3. The upper sleeve section 3 of the handle body 2 includes the holding portion 23 located closer to the distal end of the upper sleeve section 3 and the manipulating portion 24 located closer to the proximal end of the upper sleeve section 3. A guide bore 31 is defined in the manipulating portion 24 to face the movable body 10. The guide bore 31 includes an elongated hole 32 extending along the axial direction C of the blade sleeve 5 and an engagement recess 33 extending circumferentially from the proximal end of the elongated hole 32. The manipulation body 11 projects from the shaft portion 29 of the movable body 10 and is exposed from the guide bore 31 to the exterior.

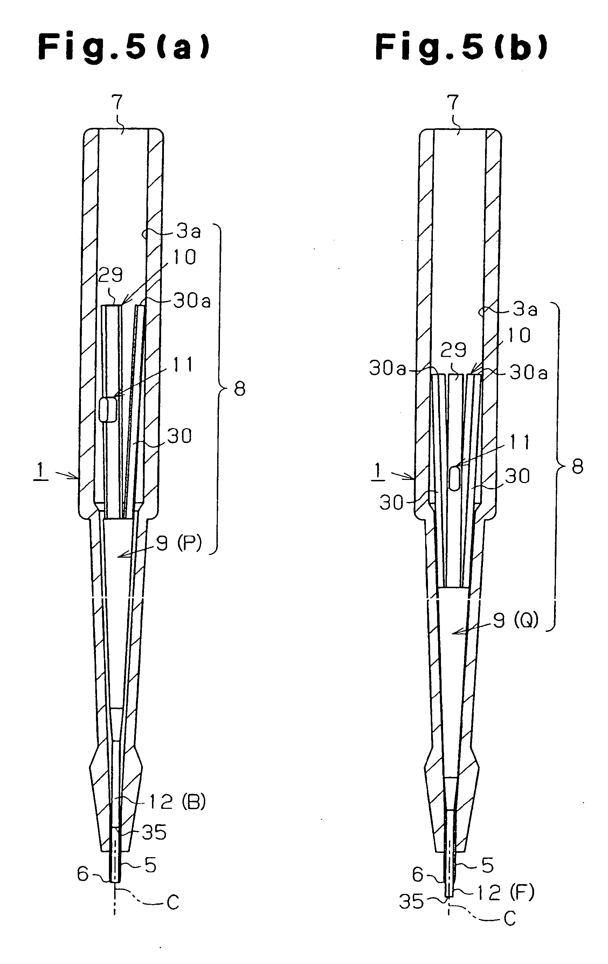

When the pressing body 9 is held in the retracted state P of Figs. 4(a) and 5(a), the manipulation body 11 is engaged with the engagement recess 33 of the guide bore 31 and held in contact with an inner wall 33a of the engagement recess 33 on the movement path of the movable body 10. The contact portion 12 of the pressing body 9 is retracted inward from the cutting edge 6 of the blade sleeve 5 and maintained at a retracted position B spaced from the cutting edge 6.

In this retracted state P, if the manipulation body 11 is revolved in a circumferential direction, the manipulation body 11 is disengaged from the engagement recess 33 of the guide bore 31. The manipulation body 11 is then moved along the guide bore 31 toward the distal end of the upper sleeve section 3, as shown in Fig. 4(b). The pressing member 8, as a whole, is thus moved against the contact resistance force of the leaf springs 30 of the pressing body 9 applied to the inner wall 3a of the upper sleeve section 3. The manipulation body 11, as illustrated in Figs. 4(c) and 5(b), is stopped when held in contact with the distal end of an inner wall 32a of the elongated hole 32.

In this state, or the projected state Q, the contact portion 12 of the pressing body 9 is held at the projected position F, or projected outwardly from the cutting edge 6 of the blade sleeve 5, by the contact resistance force of the leaf springs 30 of the pressing body 9 applied to the inner wall 3a of the upper sleeve section 3. In this projected state Q, if the manipulation body 11 is moved along the elongated hole 32 of the guide bore 31 toward the proximal end of the upper sleeve section 3, the manipulation body 11 is engaged with the engagement recess 33 of the guide bore 31. The retracted state P is thus restored.

In the second embodiment, the manipulation body 11 forms an engagement portion and a disengagement portion of a retracted position holding member. The inner wall 33a of the engagement recess 33 forms an engagement portion of the retracted position holding member. The inner wall 3a of the upper sleeve section 3 forms a pressing contact portion serving as a projected position holding member. Each of the leaf springs 30 also forms a projected position holding member.

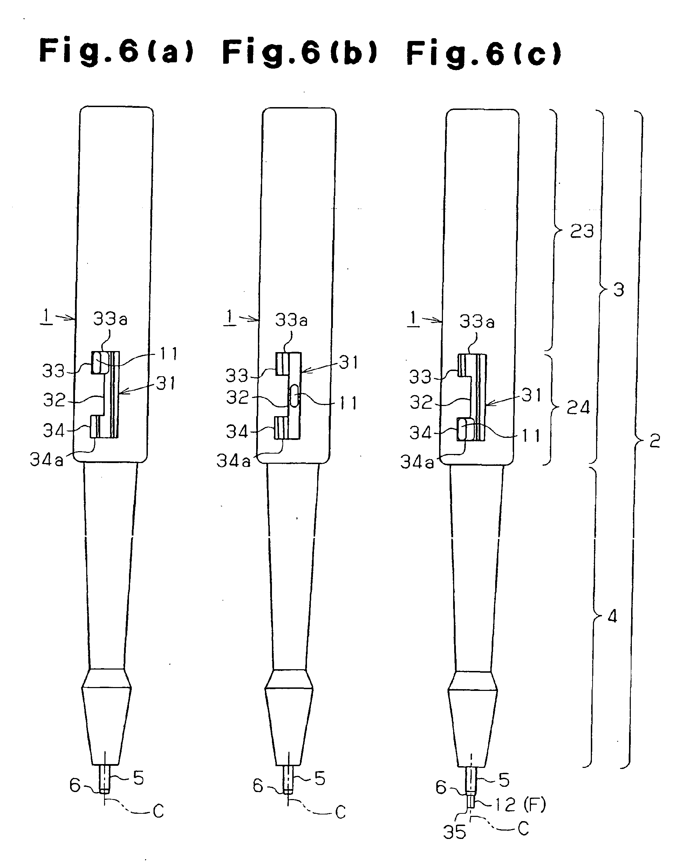

Next, a modification of the skin punch instrument according to the second embodiment will be explained with reference to Figs. 5(a), 5(b), and 6(a) to 6(c). The explanation will focus on the modified points from the second embodiment.

In addition to the engagement recess 33 defined at the proximal end of the elongated hole 32, the guide bore 31 includes an engagement recess 34 extending circumferentially from the distal end of the elongated hole 32. When the manipulation body 11 is engaged with the engagement recess 34 of the guide bore 31 and contacts an inner wall 34a of the engagement recess 34 on the movement path of the movable body 10, the pressing body 9 is switched to the projected state Q. That is, the contact portion 12 of the pressing body 9 is projected outwardly from the cutting edge 6 of the blade sleeve 5 and maintained at the projected position F. In this projected state Q, the manipulation body 11 is revolved in a circumferential direction and disengaged from the engagement recess 34. The manipulation body 11 is then moved along the elongated hole 32 toward the proximal end of the upper sleeve section 3, such that the retracted state P is restored.

In this modification, the manipulation body 11 forms an engagement portion and a disengagement portion of a projected position holding member. The inner wall 34a of the engagement recess 34 forms an engagement portion of the projected position holding member.

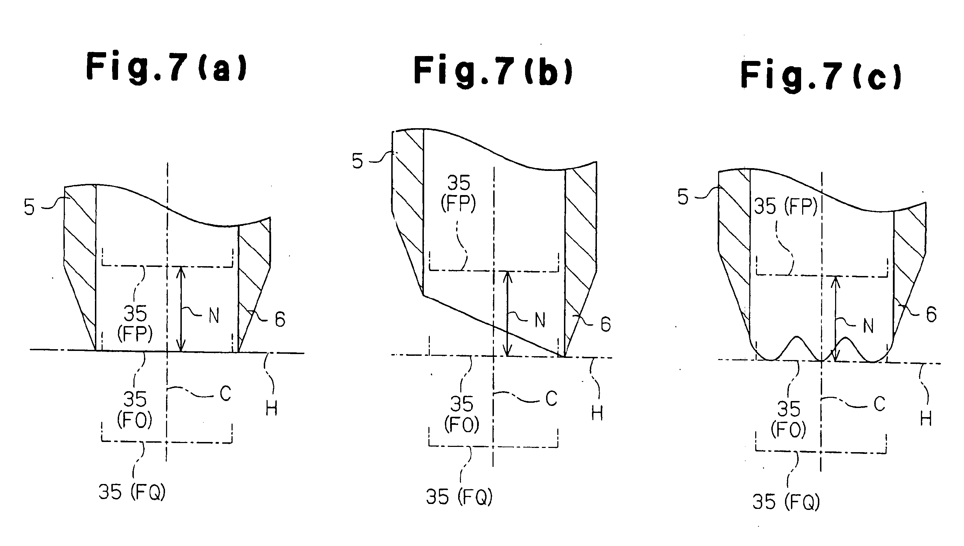

In each of the first and second embodiments and the modification of the second embodiment, the cutting edge 6 of the blade sleeve 5 is formed along a hypothetical plane H perpendicular to the axial direction C of the blade sleeve 5, as illustrated in Fig. 7(a). However, referring to Fig. 7(b), the cutting edge 6 may be inclined with respect to the hypothetical plane H. Alternatively, the cutting edge 6 may be formed in a wave-like form, as shown in Fig. 7(c). Further, a cutting surface 6a may be formed along the outer circumference of the cutting edge 6, in addition to the inner circumference.

As has been described, the contact portion 12 of the pressing body 9 is moved from the retracted position B to the projected position F, or toward the cutting edge 6 of the blade sleeve 5. In the case of the cutting edge 6 of Fig. 7(a), the "projected position F" corresponds to a projected position FQ at which a distal end surface 35 of the contact portion 12 is projected outward from the hypothetical plane H including the cutting edge 6, a reference position FO at which the distal end surface 35 corresponds to the plane H, and a retracted position FP at which the distal end surface 35 is retracted inward from the plane H. If the distal end surface 35 of the contact portion 12 is located at the projected position FQ or the reference position FO, the sample SE is easily removed from the contact portion 12. However, the distal end surface 35 may be located at the retracted position FP. In this case, it is preferred that the retraction amount N between the retracted position FP and the hypothetical plane H is substantially equal to the thickness of the sample SE such that the sample SE is easily removed from the distal end surface 35 of the contact portion 12. Particularly, it is more preferred that the retraction amount N is slightly smaller than the thickness of the sample SE, such that the sample SE is projected slightly outward from the cutting edge 6.

In the case of the cutting edge 6 of Fig. 7(b), the hypothetical plane H is defined as a plane including the outermost portion of the slanted portion of the cutting edge 6. In the case of the cutting edge 6 of Fig. 7(c), the hypothetical plane H is defined as a plane including the outermost portion of the wave-form portion of the cutting edge 6.

The advantages of the illustrated embodiments, together with the characteristics thereof, will hereafter be described.
(1) In the skin punch instrument of the illustrated embodiments, the blade sleeve 5 having the annular cutting edge 6 is formed at the distal end of the handle 1. The pressing body 9 is received in the blade sleeve 5 such that the pressing body 9 is movable in the axial direction C. The pressing body 9 is thus allowed to be located at the retracted position B at which the contact portion 12, which is formed at the distal end of the pressing body 9, is separated inwardly from the cutting edge 6 of the blade sleeve 5. The pressing body 9 is also allowed to be located at the projected position F to which the contact portion 12 is moved from the projected position F, or toward the cutting edge 6 of the blade sleeve 5. The skin punch instrument includes the manipulation members 10, 11 for moving the pressing body 9 between the retracted position B and the projected position F, as well as the projected position holding members 21, 22, 26a, 30, 3a, 11, 34a.
   Accordingly, even if the sample SE remains caught by the contact portion 12 of the pressing body 9 without separating from the contact portion 12, the pressing body 9 may be held at the projected position F such that the sample SE is easily removed from the contact portion 12.
(2) In the illustrated embodiments, the contact portion 12 of the pressing body 9 is projected outward from the cutting edge 6 of the blade sleeve 5 when located at the projected position F. Thus, at this position F, the contact portion 12, which is projected outward from the cutting edge 6, is exposed to an external object, as compared to the cutting edge 6. That is, the contact portion 12 protects the cutting edge 6 by preventing the cutting edge 6 from being interfered and damaged directly by the external object. Also, this structure further facilitates removal of the sample SE from the contact portion 12. As the gap G becomes smaller, the cutting edge 6 is further effectively prevented from being interfered and damaged by the external object.
(3) In the illustrated embodiments, the manipulation member includes the movable body 10, which is movable in the handle 1 together with the pressing body 9, and the manipulation body 11 exposed from the handle 1 to the exterior. The manipulation body 11 enables the movable body 10 to be moved together with the pressing body 9. This arrangement simplifies the structure of the manipulation member.
(4) In the first embodiment, the manipulation member includes the elastic body 16 for urging the movable body 10 such that the pressing body 9 is moved from the projected position F to the retracted position B. The movable body 10 is moved against the force of the elastic body 16 such that the pressing body 9 is moved from the retracted position B to the projected position F. The pressing body 9 is thus moved easily.
(5) In the illustrated embodiments, the engagement portion 21, 11 is provided in the movable body 10 and the engagement portion 26a, 34a is provided in the handle 1. Each of the engagement portions 21, 11, 26a, 34a functions as the aforementioned projected position holding member. The engagement portion 21, 11 is engaged with the corresponding engagement portion 26a, 34a on the movement path of the movable body 10, thus restricting movement of the pressing body 9 from the projected position F to the retracted position B. Also, the disengagement portion 22, 11 is provided in the movable body 10 for disengaging the engagement portion 21, 11 of the movable body 10 from the engagement portion 26a, 34a of the handle 1. The projected position holding member thus has a relatively simple configuration.
(6) In the second embodiment, the pressing contact portion 30 is provided in the movable body 10 and the pressing contact portion 3a is formed in the handle 1. This structure generates the contact resistance force of the movable body 10 applied to the handle 1, thus restricting the movement of the pressing body 9 from the projected position F to the retracted position B. When the movable body 10 is moved, the movement may be brought about against the contact resistance force of the movable body 10. The structure of the projected position holding member is thus simplified.
(7) In the illustrated embodiments, the retracted position holding members 21, 22, 27a, 11, 33a are provided for maintaining the pressing body 9 at the retracted position B. This structure facilitates the operation for moving the pressing body 9.
(8) In the illustrated embodiments, the pressing body 9 and the movable body 10 are received in the interior of the handle 1 through the opening 7 defined at the proximal end of the handle 1. Further, as the retracted position holding members, the engagement portion 21, 11 is provided in the movable body 10 and the engagement portion 27a, 33a is provided in the handle 1. The engagement portion 21, 11 is engaged with the corresponding engagement portion 27a, 33a on the movement path of the movable body 10 for preventing the pressing body 9 and the movable body 10 from falling from the opening 7 of the handle 1 to the exterior. The movable body 10 also includes the disengagement portion 22, 11 for disengaging the engagement portion 21, 11 of the movable body 10 from the engagement portion 27a, 33a of the handle 1. The structure of the retracted position holding member is thus relatively simple.
(9) In the first embodiment, the engagement portion 21 and the disengagement portion 22 of the movable body 10 are formed in the elastic body 20, which is provided in the movable body 10. The elastic body 20 urges the engagement portion 21 of the movable body 10 for contacting the engagement portions 26a, 27a of the handle 1. When the disengagement portion 22 is manipulated against the urging force of the elastic body 20, the engagement portion 21 is disengaged from the engagement portions 26a, 27a of the handle 1. The operation for moving the pressing body 9 is thus facilitated.
(10) In the illustrated embodiments, with the inner circumferential portion 5a of the blade sleeve 5 including the cutting edge 6 and the outer circumferential portion 12a of the contact portion 12 of the pressing body 9 held opposed to each other, the inner circumferential portion 5a and the outer circumferential portion 12a are spaced from each other by the gap G. This structure makes it possible to move the pressing body 9 smoothly with respect to the blade sleeve 5.
(11) In the illustrated embodiments, the handle 1 includes the guide bore 25, 31. The engagement portion of the movable body 10 is formed by the engagement portion 21, 11 engaged with the guide bore 25, 31. The engagement portion of the handle 1 is formed by the inner walls 26a, 27a, 33a, 34a with which the engagement portion 21, 11 is held in contact in the guide bore 25, 31. The disengagement portion of the movable body 10 is formed by the manipulating projection 22, 11 exposed from the guide bore 25, 31 to the exterior of the handle 1. The manipulation member thus has a relatively simple structure.
(12) In the illustrated embodiments, the pressing body 9, the movable body 10, and the manipulation body 11 are formed as one body for prohibiting relative movement with respect to one another. The manipulation member is thus configured relatively simple.
(13) In the first embodiment, the movable body 10 includes the spring support 13, which extends integrally from the pressing body 9, and the connecting portion 14 for connecting the spring support 13 with the manipulation body 11 as one body. The manipulation body 11 is exposed from the opening 7 defined at the proximal end of the handle 1 to the exterior. The spring support 13 of the movable body 10 supports the spring 16 with respect to the handle 1. The spring 16 urges the movable body 10 such that the pressing body 9 is moved from the projected position F to the retracted position B. For moving the pressing body 9 from the retracted position B to the projected position F, the movable body 10 is moved against the urging force of the spring 16. The structure of the manipulation member is thus relatively simple.
(14) In the first embodiment, the connecting portion 14 of the movable body 10 includes the spring 20. The engagement portion 21 and the disengagement portion 22 of the movable body 10 are formed in the spring 20. The spring 20 urges the engagement portion 21 of the movable body 10 for contacting the engagement portion 26a, 27a of the handle 1. By operating the disengagement portion 22 of the movable body 10 against the elastic force of the spring 20, the engagement portion 21 of the movable body 10 is disengaged from the engagement portions 26a, 27a of the handle 1. The manipulation member thus has a relatively simple structure.
(15) In the first embodiment, the spring provided in the connecting portion 14 corresponds to the leaf spring 20 supported in a cantilever manner. More specifically, the end of the leaf spring 20 closer to the spring support 13 of the movable body 10 corresponds to the fixed end. The end of the leaf spring 20 closer to the manipulation body 11 corresponds to the free end. The structure of the manipulation member is thus simplified.

With regard to the skin punch instruments of the first and second embodiments, and the modification of the second embodiment, it is preferred that a part or the entire part of the outer surface of the skin punch instrument is painted in a color different from the red blood color, which is, for example, a green color. In this manner, the blood is clearly visible on the outer surface of the skin punch instrument due to the difference of colors between the outer surface of the skin punch instrument and the blood.

The present invention is not restricted to the illustrated embodiments but may be embodied in the following modified forms.

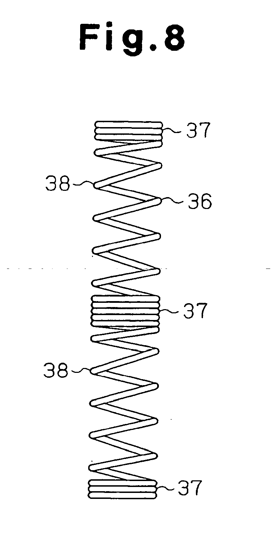

As shown in Fig. 8, the compression coil spring 16 of Fig. 1(b) may be replaced by a coil spring 36. The coil spring 36 includes densely wound portions 37 formed at opposing ends and an intermediate section. Loosely wound portions 38 are formed between adjacent ones of the densely wound portions 37. The urging force of the coil spring 36 is substantially equal to that of the compression coil spring 16. However, when stored in a storage container in a multiple number, the coil springs 36 are easily separated from each other without intertwining, as compared to the compression coil spring 16. The coil spring 36 is thus relatively easy to handle, facilitating installation of the coil spring 36 in the skin punch instrument.

Although not illustrated, a screw mechanism may be arranged between the manipulation body and the pressing body such that, by rotating the manipulation body with respect to the handle, the pressing body is moved in the axial direction of the blade sleeve. Further, the skin punch instrument may be employed for different purposes other than the removal of a skin sample, such as removal of a portion of a human organ or cornea or animal tissue.

The present examples and embodiments are to be considered as illustrative and not restrictive and the invention is not to be limited to the details given herein, but may be modified within the scope and equivalence of the appended claims.

## Claims

1. A skin punch instrument having a blade sleeve (5) including an annular cutting edge (6) formed at a distal end of a handle(1), a pressing body (9) being received in the blade sleeve (5) in an axially movable manner, wherein
a contact portion (12) is formed at a distal end of the pressing body (9), the contact portion (12) being allowed to be located at a retracted position separated inwardly from the cutting edge (6) of the blade sleeve (5) and a projected position separated from the retracted position toward the cutting edge (6) of the blade sleeve (5), the skin punch instrument comprising:
a manipulation member (11) for moving the pressing body (9) between the retracted position and the projected position; wherein the manipulation member (11) includes a movable body (10) movable in the interior of the handle (1) together with the pressing body (9) and a manipulation body (11) exposed from the handle (1) to the exterior, and wherein the movable body (10), together with the pressing body (9), is moved by means of the manipulation body (11), and wherein the manipulation member (11) includes an elastic body (16) for urging the movable body (10) such that the pressing body (9) is moved from the projected position to the retracted position, and wherein the pressing body (9) is moved from the retracted position to the projected position by moving the movable body (10) against the urging force of the elastic body (16), **characterized in that** it further comprises a projected position holding member (21, 22) for holding the pressing body (9) at the projected position,

2. The skin punch instrument according to Claim 1, wherein the contact portion (12) of the pressing body (9) is projected outwardly from the cutting edge (6) of the blade sleeve (5) at the projected position.

3. The skin punch instrument according to Claim 1, wherein the movable body (10) and the handle (1) each include an engagement portion (21) as the projected position holding member (21, 22), such that the engagement portion (21) of the movable body (10) is engaged with the engagement portion (27a) of the handle (1) on a movement path of the movable body (10) for restricting movement of the pressing body (9) from the projected position to the retracted position, and wherein the movable body (10) includes a disengagement portion (22) for disengaging the engagement portion (21) of the movable body (10) from the engagement portion (27a) of the handle (1).

4. The skin punch instrument according to claim 1, wherein the movable body (10) and the handle (1) each include a pressing contact portion (12) as the projected position holding member (21, 22) for generating a contact resistance force of the movable body (10) acting against the handle (1), such that the movement of the pressing body (9) from the projected position to the retracted position is restricted, and wherein the movable body (10) is moved against the contact resistance force of the movable body (10).

5. The skin punch instrument according to Claim 1, comprising a retracted position holding member (26, 27) for holding the pressing body (9) at the retracted position.

6. The skin punch instrument according to Claim 5, wherein the pressing body (9) and the movable body (10) are inserted into the handle (1) through an opening (7) defined in the distal end of the handle (1), wherein the movable body (10) and the handle (1) each includes an engagement portion (21, 27a) as the retracted position holding member (26, 27), such that the engagement portion (21) of the movable body (10) is engaged with the engagement portion (27a) of the handle (1) on the movement path of the movable body (10) for preventing the pressing body (9) and the movable body (10) from falling from the opening (7) defined in the handle (1), and wherein the movable body (10) includes a disengagement portion (22) for disengaging the engagement portion (21) of the movable body (10) from the engagement portion (21) of the handle (1).

7. The skin punch instrument according to Claim 3, wherein the engagement portion (21) and the disengagement portion (22) of the movable body (10) are provided in an elastic body (20) formed in the movable body (10), the elastic body (20) urging the engagement portion (21) of the movable body (10) to contact the engagement portion (27a) of the handle (1), and wherein, by operating the disengagement portion (22) of the movable body (10) against the urging force of the elastic body (20), the engagement portion (21) of the movable body (10) is disengaged from the engagement portion (27a) of the handle (1).

8. The skin punch instrument according to Claim 2, wherein the manipulation member (11) includes a movable body (10) movable in the interior of the handle (1) together with the pressing body (9) and a manipulation body (11) exposed from the handle (1) to the exterior, and wherein the movable body (10), together with the pressing body (9), is moved by means of the manipulation body (11).

9. The skin punch instrument according to Claim 4, wherein the movable body (10) and the handle (1) each include an engagement portion as the projected position holding member (21, 27a), such that the engagement portion (21) of the movable body (10) is engaged with the engagement portion (27a) of the handle (1) on a movement path of the movable body (10) for restricting movement of the pressing body (9) from the projected position to the retracted position, and wherein the movable body (10) includes a disengagement portion (22) for disengaging the engagement portion (21) of the movable body (10) from the engagement portion (27a) of the handle (1).

10. The skin punch instrument according to Claim 6, wherein the engagement portion (21) and the disengagement portion (22) of the movable body (10) are provided in an elastic body (20) provided in the movable body (10), the elastic body (20) urging the engagement portion (21) of the movable body (10) to contact the engagement portion (21) of the handle (1), and wherein, by operating the disengagement portion (22) of the movable body (10) against the urging force of the elastic body (20), the engagement portion (21) of the movable body (10) is disengaged from the engagement portion (21) of the handle (1).

## Patentansprüche

1. Ein Hautstanzgerät, welche eine Klingenhülse (5) hat, die eine ringförmige schneidende Kante (6), die an einem entfernten Ende eines Griffes (1) gebildet ist, einen pressenden Körper (9), der in der Klingenhülse (5) auf eine axial bewegliche Weise aufgenommen ist, beinhaltet, worin ein Kontaktteil (12) an einem entfernten Ende des pressenden Körpers (9) gebildet ist, dem Kontaktteil (12) ermöglicht ist, in einer eingefahrenen Position angeordnet zu sein, die innen von der schneidenden Kante (6) der Klingenhülse (5) getrennt ist, und einer ausgefahrenen Position, die von der eingefahrenen Position gegen die schneidende Kante (6) der Klingenhülse (5) getrennt ist, wobei das Hautstanzgerät umfasst: ein manipulierendes Teil (11) zu Bewegung des pressenden Körpers (9) zwischen der eingefahrenen und der ausgefahrenen Position; worin das manipulierende Teil (11) einen beweglichen Körper (10) beinhaltet, der in dem Inneren des Griffes (1) zusammen mit dem pressenden Körper (9) beweglich ist, und einen manipulierenden Körper (11), der sich vom Griff (1) nach außen erstreckt, und worin der bewegliche Körper (10), zusammen mit dem pressenden Körper (9), mittels des manipulierenden Körpers (11) bewegt wird, und worin das manipulierende Teil (11) einen elastischen Körper (16) zum Antreiben des beweglichen Körpers (10) beinhaltet, so dass der pressende Körper (9) von der ausgefahrenen Position in die eingefahrene Position bewegt wird, und worin der pressende Körper (9) von der eingefahrenen Position in die ausgefahrene Position bewegt wird, indem der bewegliche Körper (10) gegen die antreibende Kraft des elastischen Körpers (16) bewegt wird,
**dadurch gekennzeichnet, dass** es zusätzlich für das Halten des pressenden Körpers (9) in der ausgefahrenen Position ein Halteteil (21,22) für die ausgefahrene Position umfasst.

2. Hautstanzgerät nach Anspruch 1, worin der Kontaktteil (12) des pressenden Körpers (9) in der ausgefahrenen Position äußerlich zur schneidenden Kante (6) der Klingenhülse (5) ausgefahren ist.

3. Hautstanzgerät nach Anspruch 1, worin der bewegliche Körper (10) und der Griff (1) jeweils einen Eingriffsteil (21) als das Halteteil (21,22) für die ausgefahrene Position beinhalten, so dass der Eingriffsteil (21) des beweglichen Körpers (10) in den Eingriffsteil (27a) des Griffes (1) auf einem Bewegungspfad des beweglichen Körpers (10) eingreift, um die Bewegung des pressenden Körpers (9) von der ausgefahrenen Position bis zur eingefahrenen Position einzuschränken, und worin der bewegliche Körper (10) ein Abkopplungsteil (22) für das Abkoppeln des Eingriffsteils (21) des beweglichen Körpers (10) von dem Eingriffsteil (27a) des Griffes (1) beinhaltet.

4. Hautstanzgerät nach Anspruch 1, worin der bewegliche Körper (10) und der Griff (1) jeweils als Halteteil (21,22) für die ausgefahrene Position ein Presskontaktteil (12) zur Erzeugung einer Kontaktwiderstandskraft des beweglichen Körpers (10), der gegen den Griff (1) wirkt, beinhalten, so dass die Bewegung des pressenden Körpers (9) von der ausgefahrenen Position in die eingefahrene Position beschränkt ist, und worin der bewegliche Körper (10) gegen die Kontaktwiderstandskraft des beweglichen Körpers (10) bewegt wird.

5. Hautstanzgerät nach Anspruch 1, umfassend ein Halteteil (26,27) für die ausgefahrene Position zum Halten des pressenden Körpers (9) in der eingefahrenen Position.

6. Hautstanzgerät nach Anspruch 5, worin der pressende Körper (9) und der bewegliche Körper (10) in den Griff (1) durch eine Öffnung (7), die am entfernten Ende von Griff (1) definiert ist, eingefügt sind, worin der bewegliche Körper (10) und der Griff (1) jeweils als das Halteteil (26,27) für die eingefahrene Position einen Eingriffsteil (21,27a) beinhalten, so dass der Eingriffsteil (27a) des beweglichen Körpers (10) in den Eingriffsteil (27a) des Griffes (1) auf dem Bewegungspfad des beweglichen Körpers (10) eingreift, um zu verhindern, dass der pressende Körper (9) und der bewegliche Körper (10) aus der im Griff (1) definierten Öffnung (7) fallen, und worin der bewegliche Körper (10) ein Abkopplungsteil (22) zum Abkoppeln des Eingriffsteils (21) des beweglichen Körpers (10) von dem Eingriffsteil (21) des Griffes (1) beinhaltet.

7. Hautstanzgerät nach Anspruch 3, worin der Eingriffsteil (21) und der Abkopplungsteil (22) des beweglichen Körpers (10) in einem elastischen Körper (20) bereitgestellt sind, der im beweglichen Körper (10) gebildet ist, der elastische Körper (20) das Eingriffsteil (21) des beweglichen Körpers (10) antreibt, das Eingriffsteil (27a) des Griffes (1) zu kontaktieren, und worin durch Betreiben des Abkopplungsteils (22) des beweglichen Körpers (10) gegen die antreibende Kraft des elastischen Körpers (20) der Eingriffsteil (21) des beweglichen Körpers (10) vom Eingriffsteil (27a) des Griffes (1) abgekoppelt wird.

8. Hautstanzgerät nach Anspruch 2, worin das manipulierende Teil (11) einen beweglichen Körper (10) beinhaltet, der im Inneren des Griffes (1) zusammen mit dem pressenden Körper (9) und einem manipulierenden Körper (11), der sich vom Griff (1) nach Außen erstreckt, beweglich ist, und worin der bewegliche Körper (10) zusammen mit dem pressenden Körper (9) mittels des manipulierenden Körpers (11) bewegt wird.

9. Hautstanzgerät nach Anspruch 4, worin der bewegliche Körper (10) und der Griff (1) jeweils als Halteteil (21,27a) für die ausgefahrene Position einen Eingriffsteil beinhalten, so dass der Eingriffsteil (21) des beweglichen Körpers (10) in den Eingriffsteil (27a) des Griffes (1) auf einem Bewegungspfad des beweglichen Körpers (10) zur Einschränkung der Bewegung des pressenden Körpers (9) von der ausgefahrenen Position zu der eingefahrenen Position eingreift, und worin der bewegliche Körper (10) einen Abkopplungsteil (22) für die Abkopplung des Eingriffsteils (21) des beweglichen Körpers (10) vom Eingriffsteil (27a) des Griffes (1) beinhaltet.

10. Hautstanzgerät nach Anspruch 6, worin das Eingriffsteil (21) und das Abkopplungsteil (22) des beweglichen Körpers (10) in einem elastischen Körper (20) bereitgestellt sind, der in dem beweglichen Körper (10) bereitgestellt ist, der elastische Körper (20) das Eingriffsteil (21) des beweglichen Teils (21) des beweglichen Körpers (10) antreibt, den Eingriffsteil (21) des Griffes (1) zu kontaktieren, und worin, durch Betreiben des Abkopplungsteils (22) des beweglichen Körpers (10) gegen die antreibende Kraft des elastischen Körpers (20), der Eingriffsteil (21) des beweglichen Körpers (10) von dem Eingriffsteil (21) des Griffes (1) abgekoppelt wird.

## Revendications

1. Un instrument pour la perforation de la peau ayant un manchon de coupe (5) incluant un bord annulaire coupant (6) formé à l'extrémité distale d'une poignée (1), un élément comprimant (9) se déplaçant de manière axiale dans le manchon de coupe (5), où un segment de contact (12) est formé à l'extrémité distale de l'élément comprimant (9), ce segment de contact (12) pouvant se trouver en position rétractée, séparée à l'intérieur du bord coupant (6) du manchon de coupe (5) et en position prolongée, séparée de la position rétractée en direction du bord coupant (6) du manchon de coupe (5), l'instrument pour la perforation de la peau comprenant: un élément de commande (11) pour déplacer l'élément comprimant (9) entre la position rétractée et la position prolongée, où l'élément de commande (11) inclut un élément mobile (10) pouvant se déplacer à l'intérieur de la poignée (1) avec l'élément comprimant (9) et un élément de commande (11) visible de l'extérieur de la poignée (1) et où l'élément mobile (10), avec l'élément comprimant (9) est déplacé grace à l'élément de commande (11), et où l'élément de commande (11) inclut un élément élastique (16) pour pousser l'élément mobile (10) de sorte que l'élément comprimant (9) soit déplacé de la position prolongée vers la position rétractée, et où l'élément comprimant (9) est déplacé de la position rétractée vers la position prolongée en déplaçant l'élément mobile (10) contre la force élastique de l'élément élastique (16); **caractérisé en ce qu'**il comprend également un élément de maintien de la position prolongée (21, 22) pour maintenir l'élément comprimant (9) en position prolongée.

2. L'instrument pour la perforation de la peau conformément à la revendication 1, où le segment de contact (12) de l'élément comprimant (9) est projeté vers l'extérieur à partir du bord coupant (6) du manchon de coupe (5) en position prolongée.

3. L'instrument pour la perforation de la peau conformément à la revendication 1, où l'élément mobile(10) et la poignée (1) comprennent chacun une pièce d'enclenchement (21) comme élément de maintien de la position prolongée (21,22), de sorte que la pièce d'enclenchement (21) de l'élément mobile (10) est enclenchée avec la pièce d'enclenchement (27a) de la poignée (1) sur la trajectoire de l'élément mobile(10) pour restreindre le mouvement de l'élément comprimant (9) de la position prolongée vers la position rétractée, et où l'élément mobile (10) inclut une pièce de désenclenchement (22) pour désenclencher la pièce d'enclenchement (21) de l'élément mobile (10) de la pièce d'enclenchement (27a) de la poignée (1).

4. L'instrument pour la perforation de la peau conformément à la revendication 1, où l'élément mobile (10) et la poignée (1) comprennent chacun un segment de pression de contact comme l'élément de maintien de la position prolongée (21,22) pour produire une force de résistance de contact de l'élément mobile (10) agissant contre la poignée (1), de sorte que le mouvement de l'élément comprimant (9) de la position prolongée vers la position rétractée est limité et où l'élément mobile (10) est déplacé contre la force de résistance de contact de l'élément mobile (10).

5. L'instrument pour la perforation de la peau conformément à la revendication 1, comprenant un élément de maintien de la position rétractée (26, 27) pour maintenir l'élément comprimant (9) en position rétractée.

6. L'instrument pour la perforation de la peau conformément à la revendication 5, où l'élément de compression (9) et l'élément mobile (10) sont insérés dans la poignée (1) à travers une ouverture (7) réalisée dans l'extrémité distale de la poignée (1), où l'élément mobile (10) et la poignée (1) comprennent chacun une pièce d'enclenchement (21, 27a) comme l'élément de maintien de la position rétractée (26, 27), de sorte que la pièce d'enclenchement (21) de l'élément mobile (10) est enclenchée avec la pièce d'enclenchement (27a) de la poignée (1) sur la trajectoire de l'élément mobile (10) pour empêcher l'élément comprimant (9) et l'élément mobile (10) de tomber de l'ouverture (7) ménagée dans la poignée (1) et où l' élément mobile (10) comprend une pièce de désenclenchement (22) pour désenclencher la pièce d'enclenchement (21) de l'élément mobile (10) de la pièce d'enclenchement (21) de la poignée (1).

7. L'instrument pour la perforation de la peau conformément à la revendication 3, où la pièce d'enclenchement (21) et la pièce de désenclenchement (22) de l'élément mobile (10) sont disposés dans un élément élastique (20) formé dans l'élément mobile (10), l'élément élastique (20) poussant la pièce d'enclenchement (21) de l'élément mobile (10) à toucher la pièce d'enclenchement (27a) de la poignée (1) et où en maniant la pièce de désenclenchement (22) de l'élément mobile (10) contre la force de l'élément élastique (20), la pièce d'enclenchement (21) de l'élément mobile (10) est désenclenchée de la pièce d'enclenchement (27a) de la poignée (1).

8. L'instrument pour la perforation de la peau conformément à la revendication 2, où l'élément de commande (11) comprend un élément mobile (10) mobile à l'intérieur de la poignée (1) avec l'élément comprimant (9) et un élément de commande (11) visible de l'extérieur de la poignée (1), et où l'élément mobile (10), est déplacé avec l'élément comprimant (9) grâce à l'élément de commande (11).

9. L'instrument pour la perforation de la peau conformément à la revendication 4, où l'élément mobile (10) et la poignée (1) comprennent chacun une pièce d'enclenchement comme l'élément de maintien en position prolongée (21, 27a), de sorte que la pièce d'enclenchement (21) de l'élément mobile (10) est enclenchée avec la pièce d'enclenchement (27a) de la poignée (1) sur la trajectoire de l'élément mobile (10) pour limiter le mouvement de l'élément comprimant (9) de la position prolongée vers la position rétractée, et où l'élément mobile (10) comporte une pièce de désenclenchement (22) pour désenclencher la pièce d'enclenchement (21) de l'élément mobile (10) de la pièce d'enclenchement (27a) de la poignée (1).

10. L'instrument pour la perforation de la peau conformément à la revendication 6, où la pièce d'enclenchement (21) et la pièce de désenclenchement (22) de l'élément mobile (10) sont insérés dans un élément élastique (20) situé dans l'élément mobile (10), l'élément élastique (20) poussant la pièce d'enclenchement (21) de l'élément mobile (10) à toucher la pièce d'enclenchement (21) de la poignée (1), et où, en maniant la pièce de désenclenchement (22) de l'élément mobile (10) contre la force de l'élément élastique (20), la pièce d'enclenchement (21) de l'élément mobile (10) est désenclenchée de la pièce d'enclenchement (21) de la poignée (1).
